# EUROPEAN PATENT SPECIFICATION

(11) **EP 2 665 715 B1**
(45) Date of publication and mention of the grant of the patent: **26.11.2014**
(21) Application number: 11805843.7
(22) Date of filing: 22.12.2011
(51) Int. Cl.: C07D 303/04, A61K 8/00, A61Q 13/00

(54) **SPIROEPOXY MACROCYCLES AS PERFUMING INGREDIENTS**
SPIROEPOXY-MAKROCYCLEN ALS DUFTSTOFFBESTANDTEILE
MACROCYCLES SPIROÉPOXY EN TANT QU'INGRÉDIENTS PARFUMANTS

(30) Priority: 18.01.2011 WO PCT/IB2011/050203
(43) Date of publication of application: 27.11.2013
(73) Proprietor: Firmenich SA, 1211 Geneva 8 (CH)
(72) Inventor: CHAPUIS, Christian, CH-1211 Geneva 8 (CH)
(74) Representative: Carina, Riccardo Filippo
(86) International application number: PCT/EP2011/073779
(87) International publication number: WO 2012/097941

(56) References cited:
- WO-A1-2005/039521
- Kraft, Philip: "Aroma Chemicals IV: Musks" In: David J. Rowe: "Chemistry and Technology of Flavors and Fragrances", 3 March 2009 (2009-03-03), Blackwell Publishing Ltd., XP002671050, ISBN: 9781444305517 pages 143-168, compounds 4-7, 50, 53, 57, 64

## Description

### Technical field

The present invention relates to the field of perfumery. More particularly, it concerns some C₁₆-C₁₇ compounds comprising a macrocycle and a spiroepoxy moiety as defined herein below. Therefore, following what is mentioned herein, the present invention comprises the invention's compounds as part of a perfuming composition or of a perfuming consumer product.

### Prior art

In the world of perfumery, the synthetic musks family is generally recognized as composed by three main families : nitro musks, polycyclic musks and alicyclic musks (e.g. see http://en.wikipedia.org/wiki/synthetic_musk). Perfumers know that each of said families has its own distinct olfactive character and nitro musks have always been seen as a unique and highly desirable olfactive note by the person skilled in the art (a perfumer). Unfortunally, the nitro musk have been banned for health and environmental reasons, and the ideal replacer has never been found, leaving the industry with this unmet need.

To the best of our knowledge, none of the invention's compounds is mentioned in any document and strikingly said compounds possess an odor very close to the one of nitro musk.

The closest analogue known (i.e. a macrocycle having a spiroepoxy group) in the prior art to have perfuming usefulness is 1-oxaspiro[2.11]tetradecane, described in WO 2005/039521 as having a woody odor type. This document suggests to the person skilled in the art that macrocycles having a spiroepoxy group would not show any nitro musk resemblance.

The ketones corresponding to the invention's epoxides are also known musk odorants. For instance Exaltenone is described as having a musky-animal note similar to musk Tonkin (see http://www.firmenich.com/e-catalog/index.lbl). Muscenone is described in EP 584477 as having an odor with musky, slightly animal, and strong nitro character. Cosmone^{®} is described in US 6326349 as having an odor with strong musk-like nitromusk-like, powdery, fruity odor. However said ketones, even if some have nitro musk type notes, have an overall character quite different from that of the compounds of the present invention and from the nitro musks in general. Said prior art ketones could not suggest to the person skilled in the art that the invention's macrocycles having a spiroepoxy group would show any nitro musk resemblance.

### Description of the invention

We have now surprisingly discovered that a compound of formula 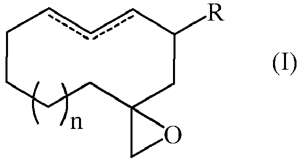
in the form of any one of its stereoisomers or a mixture thereof, and wherein one dotted line represents a single bond and the other dotted line represents a double bond;
n represents 5 or 6; and
R represents a hydrogen atom or a methyl group;
can be used as perfuming ingredient, for instance to impart odor notes very close to the nitro musk family type.

According to an embodiment of the invention, said compound (I) is one wherein R is methyl.

According to any one of the above embodiments of the invention, said compound (I) is one wherein the dotted line between the cyclanic position 5 and 6 (the spiro carbon being the 1 and the carbon bearing the R group being the 3) is a double bond and the other dotted line is a single bond.

According to any one of the above embodiments of the invention, said compound (I) is one wherein the double bond is predominantly in a configuration Z (i.e. the Z isomer represents more than 60% w/w, or more than 80%, of said compound (I), the remaining being obviously in the form of the E isomer).

According to any one of the above embodiments of the invention, said compound (I) is a C₁₆ compound.

For the sake of clarity, by the expression "any one of its stereoisomers", or the similar, it is meant the normal meaning understood by a person skilled in the art, i.e. that the invention's compound can be a pure enantiomer (if chiral) or diastereomer (e.g. the double bond is in a conformation E or Z).

For the sake of clarity, by the expression "wherein one dotted line represents a carbon-carbon single bond and the other a carbon-carbon single or double bond", or the similar, it is meant the normal meaning understood by a person skilled in the art, i.e. that the whole bonding (solid and dotted lines) between the carbon atoms connected by said dotted lines, e.g. carbon 2 and 3, is a carbon-carbon single or double bond.

As specific examples of the invention's compounds, one may cite, as non-limiting example, the three epoxides cited herein below in Table 1, containing also a description with the corresponding prior art ketones.

**Table 1 : Invention's compounds and their odor properties**

| **Structure** | **Odor** |
|---|---|
| 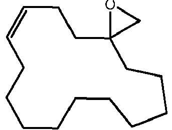 | Musk, nitro musk (reminding of the nitro musk Musk A), with a floral nuance. |
| | To the contrary of Exaltenone, only a very weak animal aspect. |
| (+-)-(6Z)-1-oxaspiro[2.14]heptadec-6-ene | No fruity notes or aspects. |
| | No woody notes or aspects. |
| 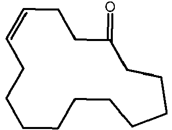 | Musky-animal, similar to musk Tonkin, pear notes. |
| | No nitro musk notes. |
| | No floral nuance. |
| Exaltenone: 4-cyclopentadecen-1-one | |
| 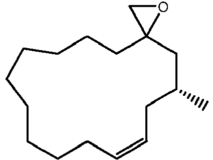 | Musk, nitro musk, notes with waxy-fatty and aldehydic/floral aspect. |
| | Fresher than corresponding ketone, and reminding the note of the nitro musk Musk K (Artander N° 2279). |
| (+)-(5R,7Z)-5-methyl-1-oxaspiro[2.14]heptadec-7-ene | No fruity notes or aspects. |
| | No woody notes or aspects. |
| 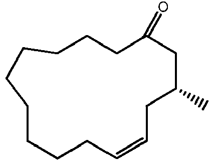 | Musky, animal, a strong nitro character. |
| | Much more animal than the corresponding epoxide. |
| Muscenone: (Z)-3-methyl-5-cyclopentadecen-1-one | |
| 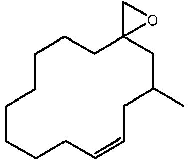 | Musk, nitro musk(reminding of the nitro musk Musk A) notes with waxy-fatty and aldehydic/floral aspect. |
| | No fruity notes or aspects. |
| | No woody notes or aspects. |
| (Z)-5-methyl-1-oxaspiro[2.13]hexadec-7-ene | Fresher than Cosmone^{®}. |
| 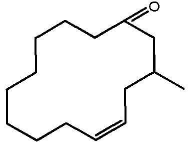 | Strong musk-like nitromusk-like, powdery, fruity odor. |
| | No floral aspects. |
| Cosmone^{®}: 3-methyl-5-cyclotetradecen-1-one | |

According to a particular embodiment of the invention, the compound of formula (I) is 1-oxaspiro[2.14]heptadec-6-ene or 5-methyl-1-oxaspiro[2.14]heptadec-7-ene or 5-methyl-1-oxaspiro[2.13]hexadec-7-ene.

As can be seen from the above Table, the odor of the invention's compounds is surprisingly of the musky type (see above the comment concerning the macrocyclic epoxide described in WO 2005/039521). Furthermore, the epoxides possess odor which are different from the ones of the corresponding ketones.

When the odor of the invention's compounds is compared with that of the corresponding prior art ketone, then the invention's compounds distinguish themselves by having a cleaner and more striking nitro musk note combined with a waxy and floral aspect, as well as by lacking any woody or fruity notes or aspect. In all cases, the epoxy compound has an animal note much weaker compared to the corresponding ketone. In some cases, the epoxy compound is lacking, or not possessing a significant, animal note to the contrary of the corresponding ketone. In any case the epoxy compound is much fresher than the ketones, so that their uses are different.

In fact the invention's compounds impart odor notes very close to the nitro musk family type, much closer than the corresponding ketones (which in some case do not even have such note in their organoleptic properties).

All the compounds mentioned above are new, i.e. not reported in the prior art, and are therefore a further object of the present invention.

As mentioned above, the invention concerns the use of a compound of formula (I) as perfuming ingredient. In other words, it concerns a method to confer, enhance, improve or modify the odor properties of a perfuming composition or of a perfumed article, which method comprises adding to said composition or article an effective amount of at least a compound of formula (I). By "use of a compound of formula (I)" it has to be understood here also the use of any composition containing a compound (I) and which can be advantageously employed in perfumery industry.

Said compositions, which in fact can be advantageously employed as perfuming ingredients, are also an object of the present invention.

Therefore, another object of the present invention is a perfuming composition comprising:
i) as perfuming ingredient, at least one invention's compound as defined above;
ii) at least one ingredient selected from the group consisting of a perfumery carrier and a perfumery base; and
iii) optionally at least one perfumery adjuvant.

By "perfumery carrier" we mean here a material which is practically neutral from a perfumery point of view, i.e. which does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting example solvents such as dipropyleneglycol, diethyl phthalate, isopropyl myristate, benzyl benzoate, 2-(2-ethoxyethoxy)-1-ethanol or ethyl citrate, which are the most commonly used. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company).

As solid carrier one may cite, as non-limiting examples, absorbing gums or polymers, or yet encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloids : Stabilisatoren, Dickungs- und Gehermittel in Lebensmittel, Band 2 der Schriftenreihe Lebensmittelchemie, Lebensmittelqualität, Behr's VerlagGmbH & Co., Hamburg, 1996. The encapsulation is a well known process to a person skilled in the art, and may be performed, for instance, using techniques such as spray-drying, agglomeration or yet extrusion ; or consists of a coating encapsulation, including coacervation and complex coacervation techniques.

By "perfumery base" we mean here a composition comprising at least one perfuming co-ingredient.

Said perfuming co-ingredient is not of formula (I). Moreover, by "perfuming co-ingredient" it is meant here a compound, which is used in a perfuming preparation or a composition to impart a hedonic effect. In other words such a co-ingredient, to be considered as being a perfuming one, must be recognized by a person skilled in the art as being able to impart or modify in a positive or pleasant way the odor of a composition, and not just as having an odor.

The nature and type of the perfuming co-ingredients present in the base do not warrant a more detailed description here, which in any case would not be exhaustive, the skilled person being able to select them on the basis of his general knowledge and according to intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

An invention's composition consisting of at least one compound of formula (I) and at least one perfumery carrier represents a particular embodiment of the invention as well as a perfuming composition comprising at least one compound of formula (I), at least one perfumery carrier, at least one perfumery base, and optionally at least one perfumery adjuvant.

It is useful to mention here that the possibility to have, in the compositions mentioned above, more than one compound of formula (I) is important as it enables the perfumer to prepare accords, perfumes, possessing the odor tonality of various compounds of the invention, creating thus new tools for his work.

For the sake of clarity, it is also understood that any mixture resulting directly from a chemical synthesis, e.g. a reaction medium without an adequate purification, in which the compound of the invention would be involved as a starting, intermediate or end-product could not be considered as a perfuming composition according to the invention as far as said mixture does not provide the inventive compound in a suitable form for perfumery. Thus, unpurified reaction mixtures are generally excluded from the present invention unless otherwise specified.

Furthermore, the invention's compound can also be advantageously used in all the fields of modern perfumery, i.e. fine or functional perfumery, to positively impart or modify the odor of a consumer product into which said compound (I) is added. Consequently, a perfuming consumer product which comprises:
i) as perfuming ingredient, at least one compound of formula (I), as defined above; and
ii) a perfumery consumer base;
is also an object of the present invention.

The invention's compound can be added as such or as part of an invention's perfuming composition.

For the sake of clarity, it has to be mentioned that, by "perfuming consumer product" it is meant a consumer product which is expected to deliver at least a perfuming effect, in other words it is a perfumed consumer product. For the sake of clarity, it has to be mentioned that, by "perfumery consumer base" we mean here the functional formulation, as well as optionally additional benefit agents, corresponding to a consumer product which is compatible with perfuming ingredients and is expected to deliver a pleasant odor to the surface to which it is applied (e.g. skin, hair, textile, or home surface). In other words, a perfuming consumer product according to the invention comprises the functional formulation, as well as optionally additional benefit agents, corresponding to the desired consumer product, e.g. a detergent or an air freshener, and an olfactive effective amount of at least one invention's compound.

The nature and type of the constituents of the perfumery consumer base do not warrant a more detailed description here, which in any case would not be exhaustive, the skilled person being able to select them on the basis of his general knowledge and according to the nature and the desired effect of said product.

Non-limiting examples of suitable perfumery consumer base can be a perfume, such as a fine perfume, a cologne or an after-shave lotion; a fabric care product, such as a liquid or solid detergent, a fabric softener, a fabric refresher, an ironing water, a paper, or a bleach; a body-care product, such as a hair care product (e.g. a shampoo, a coloring preparation or a hair spray), a cosmetic preparation (e.g. a vanishing cream or a deodorant or antiperspirant), or a skin-care product (e.g. a perfumed soap, shower or bath mousse, oil or gel, or a hygiene product); an air care product, such as an air freshener or a "ready to use" powdered air freshener; or a home care product, such as a wipe, a dish detergent or hard-surface detergent.

Some of the above-mentioned consumer product bases may represent an aggressive medium for the invention's compound, so that it may be necessary to protect the latter from premature decomposition, for example by encapsulation or by chemically bounding it to another chemical which is suitable to release the invention's ingredient upon a suitable external stimulus, such as an enzyme, light, heat or a change of pH.

The proportions in which the compounds according to the invention can be incorporated into the various aforementioned articles or compositions vary within a wide range of values. These values are dependent on the nature of the article to be perfumed and on the desired organoleptic effect as well as the nature of the co-ingredients in a given base when the compounds according to the invention are mixed with perfuming co-ingredients, solvents or additives commonly used in the art.

For example, in the case of perfuming compositions, typical concentrations are in the order of 0.001 % to 50 % by weight, or even more, of the compounds of the invention based on the weight of the composition into which they are incorporated. Concentrations lower than these, such as in the order of 0.01% to 5% by weight, can be used when these compounds are incorporated into perfumed articles, percentage being relative to the weight of the article.

The invention's compounds can be prepared according to a method as described in the examples, starting from the known ketone.

### Examples

The invention will now be described in further detail by way of the following examples, wherein the abbreviations have the usual meaning in the art, the temperatures are indicated in degrees centigrade (°C); the NMR spectral data were recorded in CDCl₃ (if not stated otherwise) with a 360 or 400 MHz machine for ¹H and ¹³C, the chemical shifts δ are indicated in ppm with respect to TMS as standard, the coupling constants J are expressed in Hz.

### Example 1

### Synthesis of compounds of formula (I)

*General procedure for oxiranylation:* to a suspension of *^{t}*BuOK (2.8g, 25 mmol) and trimethylsulfoxonium iodide (5.5g, 25 mmol) in *^{t}*BuOK (42 ml) at 50°, the macrocyclic ketone (21 mmol) was added dropwise and the mixture was kept at 50° for 6 to 18 hrs. After completion, the cold solution was diluted with Et₂O, washed twice with saturated aqueous NaHCO₃, then washed to neutrality with brine. The organic phase was dried (MgSO₄), concentrated and purified by CC (SiO₂, cyclohexane/ethyl acetate 9:1).

### I) Preparation of (6Z)-1-oxaspiro[2.14]heptadec-6-ene :

Obtained in 78% yield according to procedure after bulb-to-bulb distillation.
B.p. 120°/0.21 mbar.
¹H-NMR : 5.46-5.33 (*m,* 2H) ; 2.60 (*d, J* = 5*,* 1H); 2.57 (*d*, *J* = 5*,* 1H); 2.21-2.14 (m*,* 2H); 2.09-1.97 (*m,* 2H); 1.70-1.57 (*m,* 2H); 1.48-1.33 (*m,* 18H).
¹³C-NMR: 130.6 (*d*); 128.9 (*d*); 59.4 (*s*); 53.8 (*t*); 35.0 (*t*); 33.5 (*t*); 28.1 (*t*); 27.2 (*t*); 27.0 (*t*); 26.8 (*t*); 26.5 (*t*); 26.4 (*2t*); 26.2 (*t*); 23.4 (*t*) ; 23.2 (*t)*.

### II) Preparation of (+)-(5R,7Z)-5-methyl-1-oxaspiro[2.14]heptadec-7-ene :

Obtained in 60% yield according to procedure after bulb-to-bulb distillation as a 60:40 mixture of stereoisomers.
B.p. 120°/0.14 mbar.
α_{D}²⁰ = +15.1 neat.
¹H-NMR: (deduced from the mixture):
*Major:* 5.57-5.41 (*m*, 2H); 2.55 (*d, J* = 4.5, 1H); 2.50 (*d, J* = 4.5, 1H); 2.26-2.12 (*m*, 2H); 1.97-1.88 (*m,* 1H); 1.75-1.62 (*m,* 2H); 1.49-1.26 (*m*, 18H); 1.00 (*d, J* = 7, 3H);
*Minor:* 5.57-5.41 (*m,* 2H); 2.58 (*s,* 2H); 2.26-2.12 *(m,* 2H); 1.97-1.88 (*m*, 1H); 1.75-1.62 (*m*, 2H); 1.49-1.26 (*m*, 18H); 0.96 (*d, J* = 7, 3H).
¹³C-NMR: (deduced from the mixture) :
*Major :* 131.6 (*d*); 128.0 (*d*); 58.6 (*s*); 53.1 (*t*); 41.8 (*t*); 34.5 (*t*); 33.4 (*t*); 32.0 (*d*); 28.0 (*t*); 27.2 (*t*); 27.0 (*t*); 26.6 (*t*); 26.3 (2*t*); 26.0 (*2t*); 22.8 (*t*); 20.1 (*q*);
*Minor :* 131.8 (*d*); 128.0 (*d*); 58.7 (*s*); 53.9 (*t*); 41.8 (*t*); 34.9 (*t*); 33.4 (*t*); 32.2 (*d*); 28.1 (*t*); 27.3 (*t*); 27.0 (*t*); 26.7 (*t*); 26.2 (*t*); 26.1 (*t*); 26.0 (*t*); 25.6 (*t*); 22.3 (*t*); 19.8 (*q*).

### III) Preparation of a mixture of (Z)-5-methyl-1-oxaspiro[2.13]hexadec-7-ene :

Obtained in 52% yield according to procedure after bulb-to-bulb distillation as a 80:20 mixture of stereoisomers.
B.p. 120°/0.12 mbar.
¹H-NMR: 5.45-5.36 (*m,* 1H); 5.25-5.20 (*m,* 1H); 2.45 (*s,* 2H); 2.16-2.07 (*m,* 4H); 1.68-1.58 (*m*, 2H); 1.52-1.33 (*m*, 11H); 1.29-1.10 (*m*, 4H); 1.06 (*d, J* = *7,* 3H).
¹³C-NMR: 132.0 (*d*); 130.9 (*d*); 58.5 (*s*); 53.4 (*t*); 41.6 (*t*); 37.8 (*t*); 33.3 (*t*); 30.5 (*d*); 30.2 (*t*); 27.7 (*t*); 27.1 (*t*); 24.7 (*t*); 24.1 (*t*); 23.4 (*t*); 23.2 (*t*); 21.6 (*q*).

### Example 2

### Preparation of a perfuming composition

A perfuming composition was prepared by admixing the following ingredients:

| Parts by weight | Ingredient |
|---|---|
| 150 | Benzyl acetate |
| 20 | Geranyl acetate |
| 10 | Aldehyde C 11 lique |
| 400 | Hexylcinnamic aldehyde |
| 20 | Ambrox^{®1)} |
| 20 | Gamma undecalactone |
| 20 | Methyl benzoate |
| 200 | Bergamot essential oil |
| 50 | Citronellol |
| 100 | Coumarine |
| 10 | 10%* Damascenone |
| 30 | Estragol |
| 400 | Hedione^{®2)} HC |
| 1200 | Florol^{®3)} |
| 50 | 1,3-Benzodioxole-5-carbaldehyde |
| 250 | ISO E^{®4)} Super |
| 250 | Alpha-iso-methyl ionone |
| 350 | Linalool |
| 50 | Crystal moss |
| 800 | Hedione^{®5)} |
| 350 | Phenethylol |
| 20 | Polysantol^{®6)} |
| 50 | Vanilline |
| 100 | Violets essential oil |
| 100 | Ylang essential oil |
| 5000 | |

| | |
|---|---|
| * in dipropyleneglycol 1) (-)-(8R)-8,12-epoxy-13,14,15,16-tetranorlabdane; origin: Firmenich SA, Geneva, Switzerland 2) methyl cis-dihydrojasmonate; origin: Firmenich SA, Geneva, Switzerland 3) tetrahydro-2-isobutyl-4-methyl-4(2H)-pyranol; origin: Firmenich SA, Geneva, Switzerland 4) 1-(octahydro-2,3,8,8-tetramethyl-2-naphthalenyl)-1-ethanone; origin: International Flavors & Fragrances, USA 5) methyl dihydrojasmonate; origin: Firmenich SA, Geneva, Switzerland 6) 3,3-dimethyl-5-(2,2,3-trimethyl-3-cyclopenten-1-yl)-4-penten-2-ol; origin: Firmenich SA, Geneva, Switzerland | |

The addition of 5000 parts by weight of (6Z)-1-oxaspiro[2.14]heptadec-6-ene (as obtained in example 1.I) to the above-described composition imparted to the latter a clear nitro musk note, reminiscent of Musk Ambrette (see Arctander N°2278), as well as a nice floral nuance. However, when to the above-described composition was added the same amount of its ketone equivalent (Exaltenone), the result was significantly different and the composition acquired a clear musk, fecal/animal and fruity connotation absent in the composition obtained with the invention's epoxide.

The addition of 5000 parts by weight of (+)-(5R,7Z)-5-methyl-1-oxaspiro[2.14]heptadec-7-ene (as obtained in example 1.II) to the above-described composition imparted to the latter a clear nitro musk note as well as a nice fresh aldehydic/floral nuance. However, when to the above-described composition was added the same amount of its ketone equivalent (Muscenone), the result was significantly different and the composition acquired a clear musk note, with the nitro musk character much weaker that the previous composition, as well as an animal/powdery connotation absent in the composition obtained with the invention's epoxide.

The addition of 5000 parts by weight of a mixture of (Z)-5-methyl-1-oxaspiro[2.13]hexadec-7-ene (as obtained in example 1.III) to the above-described composition imparted to the latter a clear nitro musk note, reminiscent of Musk Ambrette, as well as a nice fresh aldehydic/floral nuance. However, when to the above-described composition was added the same amount of its ketone equivalent (Muscenone), the result was significantly different and the composition acquired a clear musk note, with the nitro musk character much weaker that the previous composition, as well as an animal/powdery and fruity connotations absent in the composition obtained with the invention's epoxide.

## Claims

1. Use as perfuming ingredient of a compound of formula (I), 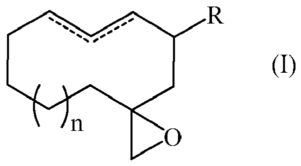
in the form of any one of its stereoisomers or a mixture thereof, and wherein one dotted line represents a single bond and the other dotted line represents a double bond;
n represents 5 or 6; and
R represents a hydrogen atom or a methyl group.

2. Use according to claim 1, **characterized in that** in said compound (I) R is methyl.

3. Use according to claim 1, **characterized in that** said compound (I) is a C₁₆ compound.

4. Use according to claim 1, **characterized in that** said compound (I) is 1-oxaspiro[2.14]heptadec-6-ene or 5-methyl-1-oxaspiro[2.14]heptadec-7-ene or 5-methyl-1-oxaspiro[2.13]hexadec-7-ene.

5. A compound of formula (I), as defined in any one of claims 1 to 4.

6. A perfuming composition comprising
i) at least one compound of formula (I), as defined in any one of claims 1 to 3;
ii) at least one ingredient selected from the group consisting of a perfumery carrier and a perfumery base; and
iii) optionally at least one perfumery adjuvant.

7. A perfuming consumer product comprising:
i) at least one compound of formula (I), as defined in any one of claims 1 to 5; and
ii) a perfumery consumer base.

8. A perfuming consumer product according to claim 7, **characterized in that** the perfumery consumer base is a perfume, a fabric care product, a body-care product, an air care product or a home care product.

9. A perfuming consumer product according to claim 8, **characterized in that** the perfumery consumer base is a fine perfume, a cologne, an after-shave lotion, a liquid or solid detergent, a fabric softener, a fabric refresher, an ironing water, a paper, a bleach, a shampoo, a coloring preparation, a hair spray, a vanishing cream, a deodorant or antiperspirant, a perfumed soap, shower or bath mousse, oil or gel, a hygiene product, an air freshener, a "ready to use" powdered air freshener, a wipe, a dish detergent or hard-surface detergent.

## Patentansprüche

1. Verwendung als parfümierender Bestandteil einer Verbindung der Formel (I) 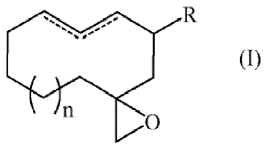
in der Form von einem ihrer Stereoisomere oder einem Gemisch davon, und wobei eine gestrichelte Linie eine einfache Bindung darstellt und die andere gestrichelte Linie eine doppelte Bindung darstellt;
n 5 oder 6 darstellt; und
R ein Wasserstoffatom oder eine Methylgruppe darstellt.

2. Verwendung gemäß Anspruch 1, **dadurch gekennzeichnet, dass** in der Verbindung (I) R Methyl ist.

3. Verwendung gemäß Anspruch 1, **dadurch gekennzeichnet, dass** die Verbindung (I) eine C₁₆-Verbindung ist.

4. Verwendung gemäß Anspruch 1, **dadurch gekennzeichnet, dass** die Verbindung (I) 1-Oxaspiro[2.14]heptadec-6-en oder 5-Methyl-1-oxaspiro[2.14]heptadec-7-en oder 5-Methyl-1-oxaspiro[2.13]hexadec-7-en ist.

5. Verbindung der Formel (I), wie in einem der Ansprüche 1 bis 4 definiert.

6. Parfümierende Zusammensetzung, umfassend
i) mindestens eine Verbindung der Formel (I), wie in einem der Ansprüche 1 bis 3 definiert;
ii) mindestens einen Bestandteil, ausgewählt aus der Gruppe, bestehend aus einem Träger für Parfümfabrikation und einer Basis für Parfümfabrikation; und
iii) wahlweise mindestens einen Hilfsstoff für Parfümfabrikation.

7. Parfümierendes Verbraucherprodukt, umfassend:
i) mindestens eine Verbindung der Formel (I), wie in einem der Ansprüche 1 bis 5 definiert; und
ii) eine Basis für Parfümfabrikation für Verbraucher.

8. Parfümierendes Verbraucherprodukt gemäß Anspruch 7, **dadurch gekennzeichnet, daß** die Basis für Parfümfabrikation für Verbraucher ein Parfüm, ein Gewebepflegeprodukt, ein Körperpflegeprodukt, ein Luftpflegeprodukt oder ein Hauspflegeprodukt ist.

9. Parfümierendes Verbraucherprodukt gemäß Anspruch 8, **dadurch gekennzeichnet, daß** die Basis für Parfümfabrikation für Verbraucher ein feines Parfüm, ein Kölnischwasser, eine Aftershavelotion, ein flüssiges oder festes Detergens, ein Weichspüler, ein Gewebeauffrischer, ein Bügelwasser, ein Papier, ein Bleichmittel, ein Shampoo, eine Farbzubereitung, ein Haarspray, eine Tagescreme, ein Deodorant oder Antiperspirant, eine parfümierte Seife, Dusch- oder Badeschaum, -öl oder -gel, ein Hygieneprodukt, ein Luftverbesserer, ein "gebrauchsfertiger" pulverförmiger Luftverbesserer, ein Wischtuch, ein Geschirrspülmittel oder Detergens für harte Oberflächen ist.

## Revendications

1. Utilisation comme ingrédient parfumant d'un composé de formule (I) 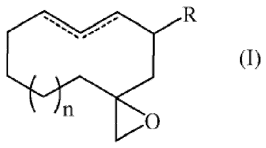
sous la forme de l'un quelconque de ses stéréoisomères ou d'un mélange de ceux-ci, et dans lequel un trait en pointillé représente une liaison simple et l'autre trait en pointillé représente une double liaison;
n représente 5 ou 6; et
R représente un atome d'hydrogène ou un groupe méthyle.

2. Utilisation selon la revendication 1, **caractérisée en ce que** dans ledit composé (I) R est un méthyle.

3. Utilisation selon la revendication 1, **caractérisée en ce que** ledit composé (I) est un composé en C₁₆.

4. Utilisation selon la revendication 1, **caractérisée en ce que** ledit composé (I) est du 1-oxaspiro[2.14]heptadéc-6-ène ou du 5-méthyl-1-oxaspiro[2.14]heptadéc-7-ène ou du 5-méthyl-1-oxaspiro[2.13]hexadéc-7-ène.

5. Composé de formule (I), tel que défini dans l'une quelconque des revendications 1 à 4.

6. Composition parfumante comprenant
i) au moins un composé de formule (I), tel que défini dans l'une quelconque des revendications 1 à 3;
ii) au moins un ingrédient choisi dans le groupe constitué d'un support de parfumerie et d'une base de parfumerie; et
iii) éventuellement, au moins un adjuvant d'usage courant en parfumerie.

7. Produit de consommation parfumant comprenant:
i) au moins un composé de formule (I), tel que défini dans l'une quelconque des revendications 1 à 5; et
ii) une base de produit de consommation de parfumerie.

8. Produit de consommation parfumant selon la revendication 7, **caractérisé en ce que** la base de produit de consommation de parfumerie est un parfum, un produit d'entretien pour textile, un produit de soin corporel, un produit de traitement de l'air ou un produit d'entretien ménager.

9. Produit de consommation parfumant selon la revendication 8, **caractérisé en ce que** la base de produit de consommation de parfumerie est un parfum fin, une eau de Cologne, une lotion après-rasage, un détergent solide ou liquide, un adoucissant pour textile, une eau de linge, une eau de repassage, un papier, un agent de blanchiment, un shampooing, une préparation colorante, une laque capillaire, une crème de jour, un déodorant ou un anti-transpirant, un savon parfumé, une mousse, une huile ou un gel de bain ou de douche, un produit d'hygiène, un désodorisant d'air ambiant, un désodorisant d'air ambiant en poudre « prêt à l'emploi », une lingette, un détergent pour la vaisselle ou un détergent pour surfaces dures.
